Europäisches Patentamt

European Patent Office

Office européen des brevets

(11) Publication number: **0 150 053**
**B1**

# (12) EUROPEAN PATENT SPECIFICATION

(45) Date of publication of patent specification: 13.06.90

(21) Application number: 85100461.4

(22) Date of filing: 17.01.85

(51) Int. Cl.⁵: **A 61 K 31/195,** A 61 K 37/18, A 61 K 9/08 // (A61K31/195, 31:045, 31:23),(A61K37/18, 31:045, 31:23)

(54) Hypocaloric low osmotic aqueous preparation for infusion.

(30) Priority: 18.01.84 US 572215

(43) Date of publication of application:
31.07.85 Bulletin 85/31

(45) Publication of the grant of the patent:
13.06.90 Bulletin 90/24

(84) Designated Contracting States:
AT BE CH DE FR GB LI NL SE

(56) References cited:
WO-A-82/03552
DE-B-1 934 317

CHEMICAL ABSTRACTS, vol. 80, no. 3, 21st January 1974, page 341, no. 13870k, Columbus, Ohio. US; C. HOFERT et al.: "Balanced parenteral feeding of premature infants" & MONATSSCHR. KINDERHEILK. 1973, 121(8), 525-530

(73) Proprietor: Oehmke, Martin, Dr.
Wiesenweg 52
D-8526 Bubenreuth (DE)

(72) Inventor: Georgieff, Michael, Dr.
Gleiwitzerstrasse 22
D-6944 Hemsbach (DE)

(74) Representative: Wuesthoff, Franz, Dr.-Ing. et al
Wuesthoff & Wuesthoff Patent- und Rechtsanwälte Schweigerstrasse 2
D-8000 München 90 (DE)

(56) References cited:
Idem
CHEMICAL ABSTRACTS, vol. 99, no. 26, 26th December 1983, page 386, no. 218617x, Columbus, Ohio, US; & JP - A - 58 162 515 (TANABE SEIYAKU CO., LTD.) 27-09-1983

Courier Press, Leamington Spa, England.

**Description**

This invention relates to a preparation for peripheral venous treatment of a patient (human or animal) who suffers from severe stress or injury, renal disease or receiving ventilatory support. More specifically, it relates to D-xylitol as the sole carbohydrate which together with amino acids and a physiological amount of electrolytes is used for peripheral vein administration and will unexpectedly preserve body protein in patients suffering severe illness, trauma, sepsis or other injury, as defined herein, when given as a hypocaloric low osmotic preparation.

A hypocaloric preparation provides 400 to 1200 kcal per day which delivers less than the patient's total needs and is usually accomplished with peripheral infusion of glucose which sometimes includes amino acids. Hypercaloric preparations provide 1500 to up to 10,000 kcal per day. The present invention is unexpectedly superior to current hypocaloric regimens of carbohydrates with or without amino acids.

The metabolic response to injury can be looked upon as an adaptation to altered endogenous requirements which is characterized by a mobilization of the body's own reserves. This metabolic response includes an increased flux of fuel, amino acids and micronutrients and a redistribution from peripheral (i.e. skeletal muscle, gut mucosa and connective tissue) to visceral organs (Waterlow, J. C.; Garlick, P. J.; Millward, D. J. Elsevier, North-Holland, Publ., Amsterdam, 1978). In a teleologic sense, this metabolic response maintains normal quantities of circulating substrates to sustain tissue homeostasis, wound healing and host defense. During intravenous administration of nutrients this preservation of a characteristic substrate milieu, which has evolved over millions of years, must be considered in order to assure optimal substrate utilization in the various organs. It is this interaction between substrates and hormones which determines whether the administered nutrients are efficiently oxidized for fuel or are stored and not readily available for energy production.

The metabolic changes associated with trauma are initiated by an increase of plasma catecholamines, which leads to an α-receptor-mediated inhibition of insulin secretion. This hypoinsulinemic state lasts for approximately 1 to 6 hours and is followed by a state of hyperinsulinemia owing in part to peripheral insulin resistance. The hyperinsulinemic state persists for 4 to 10 days. Other findings, depending upon the type and severity of injury, include elevated growth hormone levels and increased glucagon and glucocorticoid concentrations (Wilmore, D. *Surg. Clin. N. Amer.*, 56, 999, 1976). These hormonal changes lead to an increased resting energy expenditure, enhanced nitrogen excretion, hyperglycemia with an augmented glucose turnover, and an activation of fatty acid and glycerol metabolism (Clowes, G. H. A., Randall, H. T., Cha, C. J. *JPEN*, 4, 195, 1980).

The peripheral insulin resistance and elevated catabolic hormones result in a net release of amino acids, primarily from muscle and connective tissue, but also from kidney and gastro-intestinal mucosa. The increased extracellular amino acid pool is associated with an enhanced gluconeogenesis in the liver to meet the requirements of those tissues that can use only glucose. In addition, there is an increased synthesis of fibrinogen and acute phase globulins in the liver (Blackburn, G. L., Phinney, S. D., in *Surgical Physiology*, ed. Burke, J. F., Philadelphia, C. V. Mosby and Co., 1980) as well as an accelerated protein synthesis rate in the wound tissue and the cellular immune system (Gross, R. L., Newberne, P. M. *Physiol. Rev.*, 60, 188, 1980). Mobilization of fatty acids aids in fulfilling the energy requirements of the liver. Despite augmented fatty acid oxidation, fewer ketone bodies are formed to serve as an alternative energy source for heart, skeletal muscle, brain, and kidney.

During sepsis, free fatty acids are released by adipose tissue, but the liver shuttles more fatty acids away from β-oxidation and ketogenesis toward triglyceride formation. Since the liver meets its energy requirement with fatty and ketoacids and not with glucose, an energy deficit may develop. The increased hepatic lipogenesis and the central fuel deficit may induce a hepatocellular fatty metamorphosis and can lead in part to hepatic dysfunction (Beisel, W. R., Wannemacher, R. W., *JPEN*, 4, 277, 1980). Parenteral nutrition support containing glucose may accelerate this process (Blackburn, G. L., Flatt, J. P., Clowes, G. H. A., et al., *Annals of Surgery*, 177, 599, 1973) since hepatic synthesis of triglycerides from exogenously administered glucose contributes to infection-induced hyperlipidemia, as does an impaired triglyceride disposal mechanism.

The failure of ketone body production in the liver contributes to a severe energy deficit in muscle tissue, which may lead to an accelerated proteolysis. Since skeletal muscle constitutes about 40 percent of body mass, the metabolism of muscle tissue plays a major role in the mobilization and oxidation of amino acids in the periphery. Branched chain amino acids and intermediates of some dispensible amino acids are oxidized in skeletal muscle to fulfill the energy deficit and to provide nitrogen groups and carbon skeletons for the synthesis of alanine and glutamine. These two amino acids are then released at rates greater than their concentration in muscle tissue, and the increased availability of gluconeogenic substrates, mainly alanine, advances glucose production in the liver. Large quantities of intravenous glucose given as a component of parenteral nutrition therapies can be deleterious to the liver in such a situation, because they cannot reduce gluconeogenesis (Long, C. L., Jeevanandam, M., Kim, B. M., Kinney, J. M., *Am. J. Clin. Nutr.*, 30, 1340, 1977) but rather stimulate lipogenesis. The use of hypertonic carbohydrate mixtures which are exclusively for central venous administration are frequently used in clinical practice and consist of glucose, fructose, and D-xylitol in a preparation of 1:1:1 at a total dosage of 600 g/day (Georgieff, M., Geiger, K., Bratsch, H., et al. in *Recent Advances in Clinical Nutrition I*, ed. Howard, A., Baird, J., Mc.L., John Libbey and

EP 0 150 053 B1

Company, Ltd., 1981). Glucose in these solutions is reported to stimulate hepatic lipogenesis. It has been observed that using D-xylitol for hypercaloric use in hypertonic preparations is no better than using only glucose.

In liver tissue, glucose-6-P is metabolized by 4 main metabolic pathways.

$$\text{glucose} \longrightarrow \text{glucose-6-P} \begin{array}{c} \text{glycogen} \\ \uparrow \\ \\ \downarrow \\ \text{glycolysis} \end{array} \longrightarrow \text{pentose phosphate shunt}$$

After an oral glucose meal, only 20—30% of the glucose taken up by the liver is directly oxidized while the rest is converted to glycogen or triglycerides and then secreted as very low density lipoprotein-triglycerides (VLDL-TG). During continuous high carbohydrate intakes such as in currently available total parenteral nutrition regimens, the amount of glucose being metabolized in the glycolytic pathway and converted to VLDL-TG is increased, due to an activation of the enzymes involved in that process. The amount of glucose metabolized in the pentose phosphate shunt varies from 10 to over 30% of total liver glucose uptake. In this cycle ribose and deoxyribose are synthesized for the formation of nucleic acid, the initial step of protein synthesis (Newsholme, E. A., and Start, C., *Regulation in Metabolism,* John Wiley and Sons, London, New York, Sydney, Toronto, 1976). After illness the rate of glucose oxidized via the pentose phosphate shunt is more than doubled (Wannemacher, R. W., Jr., Beall, F. A., Canonico, P. G., et al., *Metabolism,* 29, 201, 1980). A large amount of glucose derived from amino acids during the process of gluconeogenesis after trauma enters this pathway thus contributing to the loss of protein.

During prolonged periods of illness, and notwithstanding conventional intravenous feeding, a significant loss of body weight is often observed. Of major concern to all in the field of critical care medicine is the loss in lean body mass, i.e. muscle, organs, etc. This loss correlates to a loss of body nitrogen. When amino acids originating from lean body mass are converted to glucose in the liver, urea is generated and excreted in the urine. The determination of urinary urea-nitrogen content can, therefore, indicate a decrease in lean body mass. By measuring nitrogen intake and output, a nitrogen balance can be made.

A nitrogen excretion exceeding the intake is often seen after trauma, sepsis, and other severe illness and can result in morbidity, even mortality. Protein depletion, particularly of visceral organs, represents the single most important unresolved aspect of trauma today. A loss of 30% of the original body weight during intravenous feeding in a seriously ill patient very often leads to death (Hadley, H. D., "Percent of Weight Loss: A Basic Indicator of Surgical Risk", *Journal American Medical Assoc.,* 106, 458, 1936 and Taylor and Keyes, "Criteria of Physical Fitness in Negative Nitrogen Balance", *Ann. N.Y. Acad. Sci.,* 73, 465, 1958).

Attempts at feeding patients intravenously are complicated because the intravenous route represents an abnormal method of administering nutrients in the body. During oral food intake, the hepatic portal vein drains most of the absorption area of the gut so that apart from long chain triglycerides, which are taken up via the thoracic lymph duct, dietary nutrients are absorbed via the portal vein (Havel, R. J., *N. Engl. J. Med.,* 287, 1186, 1972). Most compounds that are absorbed from the gut pass through the liver, and therefore, the liver is forceably situated to function as the initial regulator of the blood systemic level of many compounds that enter the body through the gut. This regulating function of the liver is especially important regarding glucose uptake and utilization. After an oral carbohydrate containing meal, glucose concentrations in the portal blood increase from about 5 to 40 mM or more while peripheral blood glucose concentrations range only from 4 to maximally 10 mM. The ability of the liver to remove glucose from the portal blood is guaranteed by an enzyme, glucokinase, which is only found in the liver.

Glucokinase activity is regulated by portal venous glucose and insulin levels. In contrast to oral food intakes, during intravenous glucose infusions, portal venous blood glucose concentrations do not reach high peak levels as after oral uptake and glucokinase activity remains relatively low (Jshida, T., Chap, Z., Chuo, J., Lewis, R., Hartley, C., et al., *J. Clin. Invest.,* 72, 590, 1983) so that liver is not capable in functioning as a buffer for glucose homeostasis. Therefore, during an intravenous glucose infusion, as is observed during standard parenteral nutrition feedings, the delicate interrelationship between substrates transported in the blood and their effect upon intermediary metabolism in various organs is determined by unphysiologically high systemic blood glucose and insulin levels. In contrast, the object of the present invention is the use of D-xylitol and amino acids to provide the patient with an energy source which is converted in the liver to glucose so that during intravenous feeding the liver can again regulate glucose homeostasis.

Although the most frequently used carbohydrate for intravenous feeding is glucose, recent studies have demonstrated that hepatic glucose output is substantially diminished by the infusion of 0.06 to 0.12 gm glucose per kilogram per hour (100—200 g/day). Intravenous administration of glucose above the endogenous synthesis rate causes only a marginal further reduction in endogenous glucose production. Under optimal conditions, only 30 percent of glucose entering the liver is directly oxidized; the remainder is converted to glycogen or fat. The percentage of glucose oxidized declines above an infusion rate of 0.12 gm per kilogram per hour (Wolfe, R. R., Allsop, J. R., Burke, J. F., *Metabolism,* 28, 210, 1979).

During intravenous infusion of 600 gm glucose together with amino acids, approximately 130 gm of triglyceride are synthesized in the liver. These triglycerides are bound to lipoproteins and released as VLDL triglycerides. The rate-limiting step during hepatic lipogenesis is not the conversion of glucose to fatty acids and the esterification to triglycerides, but the incorporation of triglycerides into the VLDL fraction. It is easily understood that continuous intravenous glucose infusion at high rates will exceed the capacity of the liver to synthesize VLDL triglycerides. The accumulation of triglycerides results in periportal fatty infiltration and a rise of liver specific enzymes.

Large intravenous doses of glucose also produce hyperglycemia and hyperinsulinemia. An increase in secretion of insulin and to a greater extent, the administration of exogenous insulin reduces muscle catabolism, thereby improving nitrogen balance. Under these circumstances, protein synthesis is shifted to the peripheral organs, mainly to muscle tissue (Woolfson, A. M. J., *N. Engl. J. Med.,* 300, 14, 1979). In the early state of trauma (4 to 7 days post-trauma), intravenous administration of glucose in excess of 0.06 g/kg BW.h (100 g/day) results in a progressive decline of total protein, albumin, pre-albumin, retinol-binding protein, and transferrin (Loehlein, D., Zick, R., *Infusionstherapie,* 8, 133, 1981) concentrations.

Because intravenously administered glucose bypasses the liver, the organ of blood glucose homeostasis, even rates of 0.12 gm per kilogram per hour are associated with a significant increase in blood glucose and insulin levels in the critically ill patient (Elwyn, D. H., Kinney, J. M., Jeevanandam et al., *Ann. Surg.,* 190, 177, 1979). Most important, however, gluconeogenesis and the concomitant loss of lean body mass is not reduced. As the gut mucosa also contributes to protein wasting by increasingly releasing amino acids after trauma, any intravenous nutritional therapy not reducing protein wasting, will simultaneously prolong the availability of adequate oral nutrient uptake, which is unquestionably the most efficient route for feeding a patient.

In contrast to the present art, a goal of this invention in feeding the critically ill intravenously is to moderate blood glucose and insulin elevations and to attenuate the loss of lean body mass by reducing gluconeogenesis. During the hypoinsulinemic state of trauma, D-xylitol is oxidized at a significantly higher rate than glucose, without extensive hyperglycemia (DeKalbermatten, N., Ravussin, E., Maeder, E., et al., *Metabolism,* 29, 62, 1980). It enters the pentose phosphate shunt directly and does not require insulin. The maximal turnover capacity is elevated after trauma. For maintenance of cellular energy levels in the skeletal muscle, a continuous supply of small amounts of glucose in necessary but this can be accomplished most safely by the infusion of D-xylitol. Maximal xylitol disposal rate in man during normal metabolic conditions is 0.37 g/kg BW.h (620 g/day). Depending on the severity of an injury, the maximal disposal rate may increase to 0.6 g/kg BW.h (1000.0 g/day) and 0.76 g/kg BW.h (1277 g/day) (Ackermann, R. H., *Infusionstherapie,* 7, 113, 1980) respectively. In contrast to D-xylitol, maximal glucose disposal rates after injury are reduced by approximately 36% from 1440 g/day to 925 g/day, even supraphysiologic insulin concentrations are not capable of increasing the limit after injury (Black, P. R., Brooks, D. C., Bessey, P. Q. et al., *Ann. Surg.,* 196, 420, 1982). Unlike glucose, intravenously administered D-xylitol is primarily metabolized in the liver and there converted to glucose independent of insulin (Pellaton, M., Acheson, K., Maeder, E. et al., *JPEN,* 2, 627, 1978).

Insulin concentrations higher than basal levels are not needed for utilization of D-xylitol when infused at low rates of 0.08—0.1 g/kg BW.h (125—185 g/day). At these infusion rates D-xylitol not only will not stimulate insulin release but also will not require insulin to be metabolized to the triose part of the glycolytic pathway. Insulin, however, is needed for further metabolism. Thus, the generation of glucose-6-phosphate in the liver cells requires far less insulin than that required for the activation of glucose.

Previous studies have shown that during long term infusion of D-xylitol alone at a rate of 0.125 g/kg BW.h (210 g/day) in healthy volunteers, blood glucose levels actually decrease, due to reduced gluconeogenesis, since during its intravenous infusion, xylitol leads to a higher glycogen deposition than glucose, and glycogen is one of the most potent inhibitors of gluconeogenesis. In addition, the concentration of insulin needed for preventing gluconeogenesis is likely to be much lower than that needed for the activation of glucose in the liver cell.

During insulin resistance, as seen following mild elective surgery, xylitol infusions reduced β-hydroxybutyrate formation to a lesser degree compared to glucose. The use of xylitol as an energy source in a dosage of 0.11 g/kg BW.h (185 g/day) was associated with significantly higher levels of FFA (Free Fatty Acids) compared to either hyper- or hypocaloric glucose infusion. Simultaneously, the levels of β-hydroxybutyrate and acetoacetate were significantly higher in patients receiving hypocaloric amounts of xylitol. These results obtained from patients with a comparable surgical procedure indicate that during the post-operative infusion of hypocaloric amounts of xylitol, endogenous fat is mobilized and oxidized more rapidly, thus contributing more to energy expenditure than during hypocaloric glucose infusions. However, nitrogen balance, an indicator of net protein catabolism was not improved with xylitol and amino acids.

At present, xylitol has either only been used as part of hypertonic carbohydrate mixture solution consisting of glucose, fructose, xylitol in a preparation of 200 g/200 g/200 g with a total intake of 600 g/per day or given to unstressed or only mildly stressed patients. The presently available xylitol and amino acid mixture solution is only to be used as a part of a hypercaloric infusion regimen. This xylitol/amino acid solution also contains electrolytes, is hypertonic, and cannot be given into a peripheral vein. The ability of a peripherally administrable xylitol and amino acid solution to preserve protein in a critically ill patient has yet not been demonstrated and in fact would be unexpected from the prior art. The use of *hypertonic* xylitol together with amino acids in elective surgery patients in contrast results in significantly higher nitrogen excretion in the urine (Georgieff, M., Kattermann, R., Geiger, K., et al., *Infusionstherapie,* 2, 69, 1981). Therefore, the art concerning xylitol and amino acid preparations suggests that such infusions would not be useful in critically ill patients.

It is the object of the present invention to provide an energy source which can be used together with amino acids for a patient obliged to receive nutritional requirements via parenteral administration to optimize the nutritional regimen. In this regard, it is an object to provide an energy source which is compatible for prolonged use in the substrate homeostasis, which results in nitrogen sparing of a patient who is suffering stress or injury and which will promote wound healing, host defense, immune competence, non-sepsis, fast resumption of oral nutrient intake, and survival of the patient. This goal is obtained through this invention, in which a novel preparation and a method of administering it is disclosed to support a critically ill patient and reduce protein wasting and gluconeogenesis by using low osmotic hypocaloric preparations of D-xylitol and amino acids which together reduce protein wasting and supporting hepatic protein synthesis and function. This combination results in an unexpected improvement of nitrogen balance in contrast to giving D-xylitol or the amino acids alone.

Statement of the Invention

The invention described herein is a novel pharmaceutical preparation for parenteral nutrition comprising an aqueous solution, a certain amount of amino acids and D-xylitol for the treatment of patients suffering severe stress or injury or significant renal disease to reduce nitrogen wasting and its accelerated gluconeogenesis. In the novel solution of the invention suitable for peripheral and central venous administration by infusion the total amino acid content is between 10—80 g/l, preferably 35 to 50 g/l, most preferably about 35 g/l and the amount of D-xylitol is between 10—80 g/l, preferably 30 to 80 g/l, most preferably about 50 g/l. This novel solution containing also a physiological amount of electrolytes is presented in a low osmotic form in which the osmolality is less than 900 mOsm, preferably between 300—900 mOsm and can be sterilized by heat without the occurence of a Maillard reaction.

The preparation for infusion is suitable for administration as a unit module and can contain in addition a lipid admixture in emulsified form, preferably triglycerides of long and/or medium chain fatty acids with particle size <1.5 μm.

Another aspect of the present invention comprises the production of the new preparation for the treatment of a critically ill patient by administration into a peripheral and central vein to reduce gluconeogenesis and protein wasting as well as to promote endogenous fat oxidation, ketogenesis and amino acid availability for hepatic secretory protein synthesis, leukocytosis and for wound healing. The new preparation can be produced by dissolving the compounds in aqua dest., adjusting the pH to a physiological value of 6,5—7, preferably 6,8 by adding a physologic acid, if necessary, then filtering the solution, filling into containers for infusion fluids and sterilizing by steam heating for about 10—20 minutes to a temperature of 120—160°C without changing the colour of the solution.

The invention described herein is based partly upon the recognition that reducing the wasting of tissue protein and gluconeogenesis are critical to effective and successful recovery. Accordingly, an increased fatty acid oxidation and ketogenesis will reduce the obligatory need to catabolize tissue protein for energy and therefore provide more precursors for wound healing, leukocytosis and hepatic secretory protein synthesis, essential for recovery.

That such a nutritional regimen could reduce nitrogen wasting and promote hepatic secretory protein synthesis, and in turn improve wound healing and recovery is not only surprising but is also a radical departure from the art. That is, the newly discovered pharmacologic effect of hypocaloric amino acid and D-xylitol solutions of the present invention, specifically that such a solution will uniquely reduce gluconeogenesis and protein wasting, is highly surprising since xylitol has always been assumed to be a non-important calorie source in hospitalized patients that can be toxic at high intakes. Indeed at intakes of greater than 210 g xylitol/day, excessive losses occur in the urine associated with increased losses of potassium which can lead to cardiac arrhythmias, metabolic alkalosis and death. Oxaloacelate deposition in kidney and brain has also been ascribed to excessive xylitol intakes, which has further led to the belief that administration of such a nutrient in appreciable quantities to the critically ill is contraindicated. Intakes of xylitol greater than 400 g/day are associated with increased uric acid production due to depletion of hepatic high energy phosphates subsequently leading to increased liver specific enzymes and bilirubin, inducive of hepatic dysfunction.

Previous investigations of amino acid and xylitol solutions have: 1) recommended xylitol intakes of less than 210 g/day, 2) recommended the addition of other carbohydrate sources (glucose, fructose, sorbitol, maltose) to be administered in a *hypercaloric* regimen via central venous injection and 3) demonstrated in mildly injured patients, no unique efficacy of xylitol solutions when compared to other carbohydrate sources. This invention is based in part on newly discovered aspects of the body's response to serious injury, involving a failure of other common intravenous nutrients to reduce protein wasting and gluconeogenesis.

The novel intravenous solution of the present invention, and its use, provide a significant advance over known infusion solutions and known nutritional therapies in reducing protein wasting and supporting recovery. While it is known in the art that xylitol and amino acids preparations of this invention have anti-catabolic properties, it is highly surprising that such a solution could reduce urinary nitrogen loss, increase fat oxidation and ketogenesis and increase hepatic protein synthesis in a critically ill patient to such a degree, and thus promote and even optimize wound healing, host defense and survival.

This invention is particularly useful for critically ill patients who require ventilatory support. The intermittent positive pressure breathing during surgery and ventilatory support can result in up to a 45% reduction in hepatic blood flow. Patients who receive continuous positive pressure ventilation especially with positive and expiratory pressure (PEEP) have extremely high rates (greater than 75%) of hepatic dysfunction secondary to reduced blood flow. This reduced blood flow coupled with standard nutrition regimens containing glucose leads inevitably to central lobular lipid deposition in the liver and serious hepatic disease.

In contrast, the novel solution and method of this invention can uniquely reduce fatty liver and subsequent hepatic dysfunction.

Efficiency is of critical importance in this regard. Although a hypertonic (only suitable for central venous administration) amino acid and xylitol preparation is currently on the market (LXA®, Boehringer-Mannheim Salvia, Mannheim, FRG) (it contains 8% amino acids, 12.5% xylitol and additional electrolytes), the product, due to its extreme hypertonicity, is limited to central venous injections and specifies on its product insert its use only with additional glucose and fructose calories to deliver all of the patient's calorie needs. Central venous catheterization in a critically ill patient is a serious surgical procedure and carries an overall complication rate of 12%. This invention teaches that such hypertonic multi-nutrient regimens to be administered by central venous injection are unnecessary in critically ill patients and that a solution of amino acids and xylitol which can be given safely through a peripheral vein can unexpectedly better support the critically ill patient. The novel formula and solution of the present invention obviates some of the adverse side effects of current nutritional therapies by moderating the blood glucose and insulin increases seen with current glucose-containing solutions which increase resting energy expenditure and promote hepatic lipogenesis. Such a novel invention also increases endogenous fat oxidation and reduces carbon dioxide production as well as lipoprotein synthesis.

In view of the limitations on the amount of nutritional support which can be provided to a patient, it is typically impossible to replace all of the normal nutritional intake of a critically ill patient. In that situation, it becomes vitally important to administer a nutritional therapy which most efficiently reduces tissue protein loss. In many smaller hospitals, pharmacies are not well equipped to manufacture special solutions to such critically ill patients, and physicians do not routinely use central venous injections for nutritional support. This invention offers a substantial benefit in that it can be manufactured and sterilized by standard techniques with an extended shelf-life without the formation of browning products due to the Maillard reaction. Such an invention would offer a substantial benefit to smaller institutions since it could be obtained in bulk from a central supplier and administered by standard peripheral vein infusion.

Another facet of the invention is the addition of a triglyceride emulsion to the amino acid and xylitol solution to be administered into a peripheral vein. Because this xylitol and amino acid solution enhances fat oxidation, simultaneously administered triglyceride will be oxidized at a faster rate providing the seriously ill patient with additional calories to reduce protein loss. By adding a triglyceride emulsion to the aqueous amino acid and xylitol preparation, the tonicity of the solution is unaffected, thereby permitting its administration into a peripheral vein. However, the additional lipid emulsion now provides the patient with sufficient calories to meet their total energy requirements in a mixture which unexpectedly reduces nitrogen loss and spares body protein.

This invention relates to the use of xylitol with amino acids in order to support protein retention in critically ill patients. The amino acids contained in this invention are all normally administered for protein synthesis, although some can be synthesized by the body. Therefore the amino acids which will normally be included are those which the body does not synthesize and which are called dietary essential amino acids. Dietary essential amino acids are defined as those which cannot be synthesized by the body and must be either orally consumed or administered. These include L-isoleucine, L-leucine, L-valine, L-tryptophan, L-phenylalanine, L-lysine, L-methionine, L-threonine and in renal disease, L-histidine.

Dietary nonessential amino acids are defined as those that can be synthesized by the body and include L-tyrosine, L-arginine, L-alanine, L-histidine, L-proline, L-serine, glycine, L-cysteine, L-cystine. These nonessential amino acids are preferably included depending on the patient. The essential and nonessential amino acids can be present also in the form of their compounds like oligopeptides, especially as di- or tripeptides.

Chiefly, the solutions according to the invention contain per liter: 30—80 g D-xylitol, 1—6 g L-isoleucine, 1—6 g L-leucine, 0.85—5 g L-valine, 0.11—0.70 L-tryptophan, 0.3—2 g L-phenylalanine, 1—6 g L-lysine acetate, 0.15—0.80 g L-methionine, 0.5—3.0 g L-threonine, 0.67—4 g L-arginine, 0.67—4 g L-alanine, 0.25—1.5 g L-histidine, 0.82—5.0 g L-proline, 0.5—3.0 L-serine, 0.67—4 g glycine, 0.003—0.02 L-cysteine $HCl.H_2O$, and if necessary a physiological acid to adjust the pH to 6,5—7. If necessary these solutions can contain also a certain amount of other amino acids, such as tyrosine and glutamic acid and the amino acids can also be in the solution in the form of their di- or tripeptides.

As used herein the patients for whom this preparation has particularly useful properties are those who are suffering severe trauma, injury, infection in which body nitrogen losses are greater than 4 grams/day or a blood glucose level greater than 120 mg/dl. Renal disease is defined as a blood creatinine greater than 1.2 mg/dl or a blood urea greater than about $30 \pm 5$ mg/dl.

The nitrogen sparing ability of hypocaloric amounts of xylitol with amino acids will help maintain adequate organ function in those tissues rendered protein depleted by conventional nutrition therapy.

It should be stated that additionally triglyceride of medium and/or long chain fatty acids in emulsified form with particle diameters of less than 1.5 μm could be present in the aqueous amino acids and xylitol containing solution to be infused into a peripheral vein as a further source of energy in the new low osmotic preparation in an amount of 2—30% by weight of the solution.

The following examples further describe the invention.

The temperature is in degrees C and g/l means grams/liter.

## Example 1

A sterile, nonpyrogenic, stable solution suitable for intravenously infusing into a peripheral or central vein of critically ill traumatized patients is prepared from pure crystalline amino acids (in the L-form) and anhydrous D-xylitol, which are dissolved in distilled water in the following concentrations:

|  | g/l | mole/l |
|---|---|---|
| D-Xylitol | 50.000 | 0.329 |
| Amino acids |  |  |
| L-Isoleucine | 4.08 | 0.031 |
| L-Leucine | 4.08 | 0.031 |
| L-Valine | 3.64 | 0.031 |
| L-Tryptophan | 0.51 | 0.0054 |
| L-Phenylalanine | 1.69 | 0.102 |
| L-Lysine acetate (base, 2.85) | 4.03 | 0.0195 |
| L-Methionine | 0.63 | 0.00422 |
| L-Threonine | 1.91 | 0.0160 |
| L-Arginine | 2.54 | 0.0146 |
| L-Alanine | 3.00 | 0.0336 |
| L-Histidine | 1.02 | 0.0066 |
| L-Proline | 3.39 | 0.0295 |
| L-Serine | 2.12 | 0.0202 |
| Glycine | 2.35 | 0.0313 |
| L-Cysteine HCl.H$_2$O (base, 0.007) | 0.015 | 0.000097 |

In the foregoing formula, the ratio of essential amino acids to total amino acids is about 58%, and the ratio of branched chain amino acids to total amino acids is about 34%.

The pH-value of this solution is adjusted to a physiologic pH of approximately 6.8 by adding citric acid, acetic acid or other physiological acids. The solution is then filtered and filled into appropriate containers for intravenous fluids and steam sterilized at 157°C for 10 minutes without any change of colour which would indicate the appearance of Maillard reactions.

Example 2

As in Example 1 the following amounts of the compounds are dissolved in distilled water.

|  | g/l | mole/l |
| --- | --- | --- |
| D-Xylitol | 50.00 | 0.02390 |
| L-Isoleucine | 3.10 | 0.02363 |
| L-Leucine | 4.40 | 0.03354 |
| L-Lysine-L-glutamate × 2 H$_2$O | 9.10 | 0.02763 |
| L-Methionine | 3.70 | 0.02480 |
| L-Phenylalanine | 3.00 | 0.01816 |
| L-Threonine | 2.00 | 0.01679 |
| L-Tryptophane | 0.90 | 0.00441 |
| L-Valine | 3.00 | 0.02561 |
| L-Arginine | 10.00 | 0.05741 |
| L-Histidine | 3.00 | 0.01933 |
| L-Alanine | 12.00 | 0.13470 |
| L-Glutamic acid | 11.00 | 0.07478 |
| Glycine | 14.00 | 0.18649 |
| L-Proline | 14.00 | 0.12160 |
| L-Serine | 6.00 | 0.05709 |
| Acetyl-L-cysteine | 0.40 | 0.00245 |
| Glycyl-tyrosine | 1.90 | 0.00851 |
| L-Lysine | 4.040 | 0.02763 |
| L-Cysteine | 0.297 | 0.00245 |
| L-Tyrosine | 1.542 | 0.00851 |
| L-Glutamic acid | 15.06 | 0.10241 |
| Citric acid | 15.06 | 0.0030 |

This solution is then filtered, filled in containers and sterilized by heating (10 min, 157°C) without any change of colour.

8

**Claims**

1. A hypocaloric low osmotic aqueous preparation for infusion comprising dietary amino acids in an amount of 10 to 80 g/l, xylitol as sole source of the carbohydrate energy and electrolytes in a dosage of physiological requirements, this preparation comprising:

|  | g/l |
| --- | --- |
| D-Xylitol | 30—80 |
| L-Isoleucine | 1—6 |
| L-Leucine | 1—6 |
| L-Valine | 0.85—5 |
| L-Tryptophan | 0.11—0.70 |
| L-Phenylalanine | 0.3—2 |
| L-Lysine acetate | 1—6 |
| L-Threonine | 0.5—3.0 |
| L-Arginine | 0.67—4 |
| L-Alanine | 0.67—4 |
| L-Histidine | 0.25—1.5 |
| L-Proline | 0.82—5.0 |
| L-Serine | 0.5—3.0 |
| L-Cysteine·HCl·H$_2$O | 0.003—0.02 |

characterized in that it contains

| L-Methionine | 0.15—0.80 g/l, |
| --- | --- |
| Glycine | 0.67—4 g/l. |

2. Preparation according to claim 1 characterized by an osmolarity between 300 and 900 mOsm.

3. Preparation according to claims 1 or 2, wherein the total amino acid concentration is between 25 and 50 g/l and the xylitol is between 30 and 80 g/l.

4. Preparation of any of claims 1 to 3 comprising emulsified long and/or medium chain triglycerides.

5. Preparation of claim 4 in which the amount of triglycerides is between 2 and 30% by weight of the solution.

6. Preparation of claims 4 and 5 wherein the lipid particles have a mean particle diameter of less than 1,5 μm.

7. Process for preparing the preparations of claims 1 to 6 characterized in that the compounds are dissolved in aqua dest. and that the resulting solution is sterilized by heat.

8. Use of the preparation of any of claims 1 to 6 for the manufacture of an infusion solution for treatment of critically ill patients suffering from severe stress or injury, renal disease or receiving ventilatory support.

9

# EP 0 150 053 B1

**Patentansprüche**

1. Hypokalorische niederosmotische wäßrige Zubereitung für Infusionen, welche Aminosäuren für die Ernährung in einer Menge von 10 bis 80 g/l, Xylitol als alleinige Quelle für Kohlenhydratenergie und Elektrolyte in einer Dosierung nach physiologischen Erfordernissen enthält und

|  | g/l |
|---|---|
| D-Xylitol | 30—80 |
| L-Isoleucin | 1—6 |
| L-Leucin | 1—6 |
| L-Valin | 0,85—5 |
| L-Tryptophan | 0,11—0,70 |
| L-Phenylalanin | 0,3—2 |
| L-Lysinacetat | 1—6 |
| L-Threonin | 0,5—3,0 |
| L-Arginin | 0,67—4 |
| L-Alanin | 0,67—4 |
| L-Histidin | 0,25—1,5 |
| L-Prolin | 0,82—5,0 |
| L-Serin | 0,5—3,0 |
| $L\text{-Cystein}\cdot HCl\cdot H_2O$ | 0,003—0,02 |

umfaßt, dadurch gekennzeichnet, daß sie

|  |  |
|---|---|
| L-Methionin | 0,15—0,80 g/l, |
| Glycin | 0,67—4 g/l |

enthält.

2. Zubereitung nach Anspruch 1, gekennzeichnet durch eine Osmolarität zwischen 300 und 900 mOsm.

3. Zubereitung nach Anspruch 1 oder 2, worin die Gesamtaminosäuren-Konzentration 25 bis 50 g/l beträgt und das Xylitol 30 bis 80 g/l ausmacht.

4. Zubereitung nach einem der Ansprüche 1 bis 3, welche emulgierte Triglyceride mit langer und/oder mittellanger Kette umfaßt.

5. Zubereitung nach Anspruch 4, worin die Menge an Triglyceriden 2 bis 30 Gew.-% der Lösung ausmacht.

6. Zubereitung nach Anspruch 4 und 5, worin die Lipidteilchen einen mittleren Teilchendurchmesser von weniger als 1,5 μm haben.

7. Verfahren zur Herstellung der Zubereitungen der Ansprüche 1 bis 6, dadurch gekennzeichnet, daß die Verbindungen in destilliertem Wasser gelöst werden und die resultierende Lösung hitzesterilisiert wird.

8. Verwendung der Zubereitung nach einem der Ansprüche 1 bis 6 zur Herstellung einer Infusionslösung zur Behandlung von kritisch kranken Patienten, die unter schwerem Streß oder schwerer Verletzung, schwerer Nierenerkrankung leiden oder Atemunterstützung empfangen.

## EP 0 150 053 B1

**Revendications**

1. Préparation aqueuse hypocalorique, faiblement osmotique pour perfusion, comprenant des acides aminés alimentaires en une quantité de 10 à 80 g/l, du xylitol comme seule source d'énergie sous forme de glucides et d'électrolytes en une posologie répondant aux besoins physiologiques, cette préparation ayant la composition suivante:

|  | g/l |
| --- | --- |
| D-Xylitol | 30—80 |
| L-Isoleucine | 1—6 |
| L-Leucine | 1—6 |
| L-Valine | 0,85—5 |
| L-Tryptophan | 0,11—0,70 |
| L-Phénylalanine | 0,3—2 |
| L-Lysine acétate | 1—6 |
| L-Thréonine | 0,5—3,0 |
| L-Arginine | 0,67—4 |
| L-Alanine | 0,67—4 |
| L-Histidine | 0,25—1,5 |
| L-Proline | 0,82—5,0 |
| L-Sérine | 0,5—3,0 |
| L-Cystéine·HCl·$H_2O$ | 0,003—0,02 |

caractérisée en ce qu'elle contient:

|  |  |
| --- | --- |
| L-Méthionine | 0,15—0,80 g/l, |
| Glycine | 0,67—4 g/l. |

2. Préparation selon la revendication 1, caractérisée par une osmolarité comprise entre 300 et 900 mOsm.

3. Préparation selon la revendication 1 ou 2, où la concentration en acides aminés totaux est comprise entre 25 et 50 g/l et celle en xylitol entre 30 et 80 g/l.

4. Préparation de l'une quelconque des revendications 1 à 3 comprenant une émulsion de triglycérides à longue chaîne et/ou à chaîne moyenne.

5. Préparation de la revendication 4 dans laquelle la quantité des triglycéridés se situe entre 2 et 30% en poids de la solution.

6. Preparation des revendications 4 et 5 où les particules lipidiques ont un diamètre moyen des particules inférieur à 1,5 μm.

7. Procédé de préparation des solutions des revendications 1 à 6, caractérisé en ce que les composés sont dissous dans l'eau distillée et que la solution obtenue est stérilisée par la chaleur.

8. Utilisation de la préparation de l'une quelconque des revendications 1 à 6 pour l'obtention d'une solution de perfusion pour le traitement de patients dans un état extrêmement grave, souffrant de stress ou de traumatisme sévère, de maladie rénale ou placés sous ventilation.